(19)

Europäisches Patentamt
European Patent Office
Office européen des brevets

(11) **EP 2 336 765 A1**

(12) **EUROPEAN PATENT APPLICATION**

(43) Date of publication:
**22.06.2011 Bulletin 2011/25**

(51) Int Cl.:
***G01N 33/00*** *(2006.01)*   ***G01N 27/12*** *(2006.01)*

(21) Application number: **09178381.1**

(22) Date of filing: **08.12.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicants:
  • **Nanocyl S.A.**
    **5060 Sambreville (BE)**
  • **Université de Bretagne Sud**
    **56100 Lorient Cedex (FR)**

(72) Inventors:
  • **Luizzi, Frédéric**
    **B-5000, NAMUR (BE)**
  • **Mezzo, Luca**
    **IT-10076, NOLE (TO) (IT)**
  • **Feller, Jean-François**
    **F-56530, QUEVEN (FR)**
  • **Castro, Mickaël**
    **F-56100, LORIENT (FR)**

(74) Representative: **pronovem**
    **Office Van Malderen**
    **Boulevard de la Sauvenière 85/043**
    **4000 Liège (BE)**

(54) **Fibre-based electrochemical sensor**

(57)   The present invention is related to a fibre-based electrochemical sensor for the detection of at least one chemical analyte vapour in a gaseous environment comprising at least one type of composite fibres, said type of composite fibres comprising a co-continuous phase blend with a specific association of a first and a second continuous polymer phase, the first polymer phase being sensitive to the chemical analyte vapour to be detected in use, wherein said first polymer phase comprises a dispersion of carbon nanotubes at a concentration above the percolation threshold and wherein the chemical analyte is soluble in said first polymer phase.

Figure 7

EP 2 336 765 A1

**Description**

**Field of the invention**

The present invention is related to a fibre-based electrochemical sensor for the detection of at least one chemical analyte vapour.

**State of the art**

Document US 5,417,100 discloses the use of conductive polymers in composites to detect the presence of solvents in the environment. The detection of the solvents is achieved by the swelling of the polymer in contact with said solvents, increasing the space between the polymeric chains. This modification of the configuration of the polymeric chains increases the resistivity of the composite. Multiple conductive polymers can be used together in one system to sense multiple solvents as disclosed by US 5,698,089.

US 5,672,297 discloses the use of a conductive filler dispersed in an isolating solvent along with swellable polymer particles. The swelling of the polymer particles induced by the environment reduces the volume of the residual free solvent and the local concentration of the filler, which reaches the percolation threshold. The swelling of the polymeric particles thus induces major changes in the electrical conductivity of the mixture.

Document US 7,342,479 discloses the use of coatings comprising carbon nanotubes as chemical sensors, using changes in electrical resistivity of the coating to determine the concentration of a particular chemical analyte. The coating described in this document uses the intrinsic resistivity variation of the carbon nanotubes embedded in a binder such as a polymer. Such chemical sensors need to be supported and are proposed in the form of coatings on dielectric substrates.

Document US 6,315,956 discloses a sensor for detecting the presence of a chemical analyte. The disclosed sensor comprises a polymer blend comprising a first and a second continuous polymer phase (co-continuous phases). The first continuous polymer phase comprises a conductive filler at a concentration above the percolation threshold, so that said first phase is electrically conductive. The second phase is insulating and selected so that the analyte to be detected is soluble in said second phase. In the presence of the analyte, the absorption of said analyte by the second phase induces the swelling of this phase, inducing a conductivity change of the macroscopic blend. Furthermore, the first polymer phase is insensitive to the analyte.

**Aims of the invention**

The present invention aims to provide a fibre-based electrochemical sensor for the detection of at least one chemical analyte that overcomes the drawbacks of the prior art.

More particularly, the present invention aims to provide a compact and self-supported fibre-based electrochemical sensor for the detection of at least one chemical analyte.

**Summary of the invention**

The present invention is related to a fibre-based electrochemical sensor for the detection of at least one chemical analyte vapour in a gaseous environment comprising at least one type of composite fibres, said type of composite fibres comprising a co-continuous phase blend with a specific association of a first and a second continuous polymer phase, the first polymer phase being sensitive to the chemical analyte vapour to be detected in use, wherein said first polymer phase comprises a dispersion of carbon nanotubes at a concentration above the percolation threshold and wherein the chemical analyte is soluble in said first polymer phase.

According to particular preferred embodiments, the invention further discloses at least one or a suitable combination of the following features:

- the Flory-Huggins interaction parameter between the chemical analyte and the first polymer phase is smaller than 5,64 $[J^{1/2} cm^{-3/2}]$.
- said carbon nanotubes are multiwall carbon nanotubes with a specific surface area between 100 and 400 $m^2/g$.
- said first polymer phase is selected from the group consisting of polycaprolactone, polylactic acid, polyethylene oxide and polymethyl metacrylate.
- said at least one chemical analyte is selected from the group consisting of aromatic solvents, preferably styrene and toluene.
- said at least one chemical analyte is selected from the group consisting of alcohols, preferably methanol.
- said at least one chemical analyte is selected from the group consisting of chlorinated solvents, preferably trichloromethane.

- said second polymer phase is a polyolefin, preferably polyethylene or polypropylene.
- said second polymer phase is polyamide.
- the first polymer phase is polycaprolactone, for the detection, in use, of tetrahydrofurane.
- the first polymer phase is polylactic acid for the detection, in use, of Styrene.
- the first polymer phase is polyethylene oxide for the detection, in use, of methanol.
- the first polymer phase is polymethyl metacrylate for the detection, in use, of trichloromethane.

The present invention further discloses a device for measuring the concentration of at least one chemical analyte comprising a fibre-based electrochemical sensor as disclosed here above.

Preferably, said device further comprises several different fibre-based electrochemical sensors as disclosed here above.

The present invention is also related to a method for detecting at least one chemical analyte comprising the steps of:

- providing a fibre-based electrochemical sensor according to any of the above-mentioned features and embodiments;
- measuring the initial electrical conductivity of the fibre-based sensor;
- bringing said fibre-based sensor into contact with at least one chemical analyte to induce a modification of the electrical conductivity of the fibres;
- measuring the modification of the resulting electrical conductivity of said fibre-based sensor and correlating said modification with the identification of the chemical analyte to be detected.

## Brief description of the drawings

Figure 1 represents the spinning process for the production of the fibres used in the fibre-based electrochemical sensor of the present invention.

Figure 2 represents a SEM analysis of a transverse section of a fibre comprising a PP / PCL blend at 50/50 wt with 3% CNT (sample 4) dispersed in the PCL phase, after extraction of the PCL phase.

Figure 3 represents a graph of the continuity ratio of PCL measured by selective extraction of PCL using acetic acid.

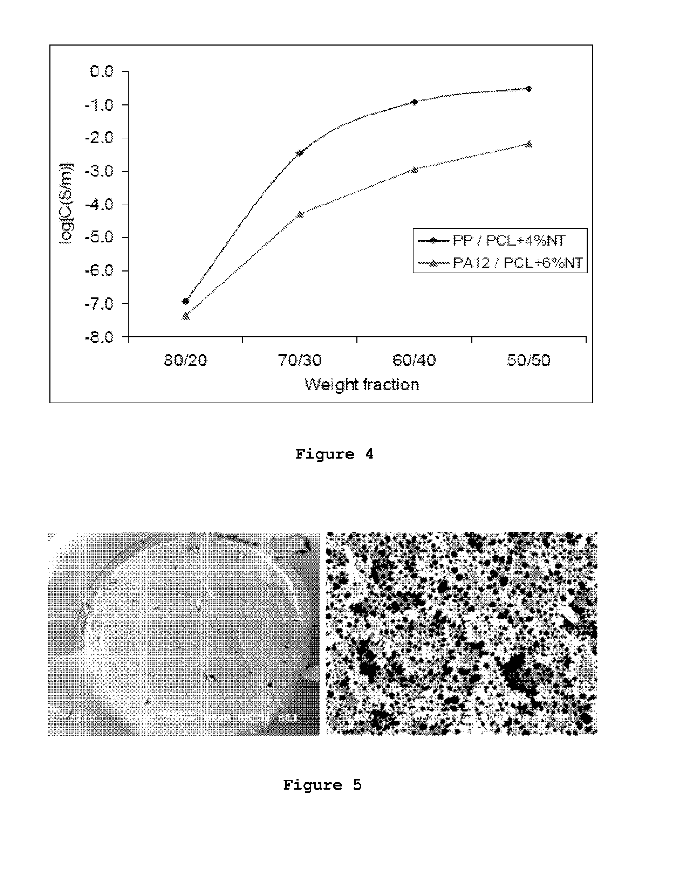

Figure 4 represents the electrical conductivity as a function of the weight fraction of PCL in both PA12 and PP.

Figure 5 represents SEM pictures of PA12/PCL blends at 50/50 wt(sample 9) after extraction of the PCL phase.

Figure 6 represents the change in electrical conductivity (relative amplitude) of fibres of various compositions in the presence of trichloromethane.

Figure 7 represents the change in electrical conductivity (relative amplitude) of fibres of various compositions in the presence of toluene.

Figure 8 represents the change in electrical conductivity (relative amplitude) of fibres of various compositions in the presence of styrene.

Figure 9 represents the change in electrical conductivity (relative amplitude) of fibres of various compositions in the presence of methanol.

Figure 10 represents the change in electrical conductivity of PA12/PMMA fibres (sample 9) in the presence of various vapours of chemical analytes in dynamic mode at 500 $cm^3$. $min^{-1}$ of vapour.

Figure 11 represents the change in electrical conductivity of PP/PCL fibres in the presence of various liquid chemical analytes.

Figure 12 represents the change in electrical conductivity of PA12/PMMA fibres (sample 9) in the presence of various amounts of vapours of chloroform in dynamic mode for various flow rates.

## Detailed description of the invention

The present invention is related to a fibre-based sensor suitable for the detection of at least one chemical analyte. The fibre sensor comprises a blend of at least two co-continuous polymer phases. By co-continuous phase blend, it is meant a phase blend comprising two continuous phases.

The first polymer phase comprises a conductive filler, such as carbon nanotubes (CNT). The chemical analyte to be detected is soluble in the first polymer phase.

By soluble, it is meant in the present invention that at least some of the chemical analytes can be absorbed or expelled by the first polymer phase in response to changes in environmental analyte concentration.

The absorption of the chemical analyte can induce a swelling of said first polymer phase. The induced swelling modifies the contact between the CNTs and therefore produces a measurable change in the electrical conductivity of the fibre.

Alternatively the diffusion of an analyte can be sufficient to induce an interaction of the chains (by Van der Waals forces or Hydrogen bonds) between the chemical analyte and said first polymer phase so that the polymer chains are able to rearrange their configuration. This will result in a movement of CNTs which will disturb the electron conduction. Thus said first polymer phase can be sensitive to poor solvents and give an electric signal even without any observable swelling of the polymer matrix.

The solubility of a solvent in a polymer can for example be deduced from the Flory-Huggins interaction parameter defined by the equation:

$$\kappa_{12} = \frac{V_{seg}\left(\delta_{Tpol} - \delta_{Tsol}\right)^2}{RT}$$

where ] $\delta_T$ is the total energy from bonds between molecules derived from:

$$\delta_T^2 = \delta_d^2 + \delta_p^2 + \delta_h^2$$

where $\delta_d$ is the energy from dispersion bonds between molecules, $\delta p$ is the energy from polar bonds between molecules and $\delta_H$ is the energy from hydrogen bonds between molecules. Examples of values of those parameters are given in table 1.

The concentration of the conductive filler in the first polymer phase is, in the absence of the analyte, above the percolation threshold, so that said first polymer phase is conductive. The percolation threshold is the minimum filler concentration at which a continuous electrically conducting path is formed in the composite. This threshold is characterised by a sharp increase of the conductivity of the blend with an increasing filler concentration. Usually, in conductive polymer composites, this threshold is considered to be the concentration of the filler which induces a resistivity below $10^6$ ohm.cm.

Table 1. Solubility parameters of solvents and polymer and Flory-Huggins interaction parameters

| | $\Delta_T$ [1] $[J^{1/2}\,cm^{-3/2}]$ | $\delta_d$ [2] $[J^{1/2}\,cm^{-3/2}]$ | $\delta_p$ [3] $[J^{1/2}\,cm^{-3/2}]$ | $\delta_h$ [4] $[J^{1/2}\,cm^{-3/2}]$ | Mol. vol. $[cm^3\,mol^{-1}]$ | $X_{12}$ [5] (PCL/solvent) | $X_{12}$ (PC/solvent) | $X_{12}$ (PMMA/solvent) | $X_{12}$ (PEO/solvent) | $X_{12}$ (PLA/solvent) |
|---|---|---|---|---|---|---|---|---|---|---|
| Water | 47.9 | 15.5 | 16 | 42.4 | 18.1 | 5.09 | 5.64 | 5.6 | 4.82 | 4.38 |
| Methanol | 29.7 | 15.1 | 12.3 | 22.3 | 40.5 | 1.10 | 1.51 | 1.48 | 0.92 | 0.65 |
| Toluene | 18.2 | 18 | 1.4 | 2 | 106.3 | 0.47 | 0.15 | 0.17 | 0.69 | 1.16 |
| Tetrahydrofuran (THF) | 19.4 | 16.8 | 5.7 | 8 | 81.4 | 0.14 | 0.02 | 0.02 | 0.26 | 0.53 |
| Chloroform | 19 | 17.8 | 3.1 | 5.7 | 79.7 | 0.2 | 0.04 | 0.05 | 0.33 | 0.62 |
| Dichloromethan | 9.9 | 8.9 | 3.1 | 3.0 | 65.3 | 3.5 | 5.64 | 2.79 | 3.99 | 4.80 |
| Styrene | 19 | 18.6 | 1 | 4.1 | 114.5 | 0.288 | 0.06 | 0.07 | 0.47 | 0.89 |
| Poly(oxyethylene) | 22.2 | 17.2 | 10.8 | 9 | 37 | – | – | – | – | – |
| Poly(lactic acid) | 23.4 | 15.4 | 15 | 9.3 | 57 | – | – | – | – | – |
| Poly(methyl methacrylate) | 20.2 | 17.02 | 5.8 | 9.2 | 84 | – | – | – | – | – |
| Poly(carbonate) | 20.1 | 18.7 | 2.9 | 7 | 203 | – | – | – | – | – |
| Poly(caprolactone) | 21.5 | 18.1 | 9 | 7.2 | 96 | – | – | – | – | – |

[1] $\delta_T$: The total energy from bonds between molecules derived from $\delta_T^2 = \delta_d^2 + \delta_p^2 + \delta_h^2$ .
[2] $\delta_d$: The energy from dispersion bonds between molecules
[3] $\delta_p$: The energy from polar bonds between molecules
[4] $\delta_H$: The energy from hydrogen bonds between molecules

[5] $\chi_{12}$: Flory-Huggins interaction parameter defined by the equation $\kappa_{12} = \frac{V_{seg}\left(\delta_{Tpol} - \delta_{Tsol}\right)^2}{RT}$

The first polymer phase is selected in such a way that it has a good compatibility with the analyte to be detected (i.e. the analyte can be absorbed). For example, in order to detect the vapour of a solvent, the first polymer phase will be selected so that the Flory Huggins interaction parameter between the analyte to be detected and the first polymer phase is smaller than about $6[J^{1/2}\,cm^{-3/2}]$ ,preferably smaller than 5,64 $[J^{1/2}\,cm^{-3/2}]$.

Due to the high compatibility of the first polymer phase with the chemical analyte, large amounts of this chemical analyte can diffuse through and be absorbed by said first polymer phase, resulting in a modification of the local mobility of the polymer chain, inducing a CNT reorganisation resulting in a modification of the macroscopic resistivity of the fibre. This diffusion may entail a swelling of the first polymer phase.

At high analyte concentrations, the swelling can be so high that the mechanical properties of the first phase severely drop. For that reason, a supporting material is necessary to maintain the mechanical integrity of the fibre. This supporting material is formed by the second polymer phase. Said second polymer phase is selected to be almost insensitive to the chemical analyte and is nonconductive. The second polymer phase also act as a protective envelope which, in use, preserves the fibre integrity.

Preferably, when used in a liquid environment, the second polymer phase is selected from the group consisting of polyolefin, more preferably polypropylene or polyethylene.

Preferably, when used in a gaseous environment, the second polymer phase is based upon polyamide.

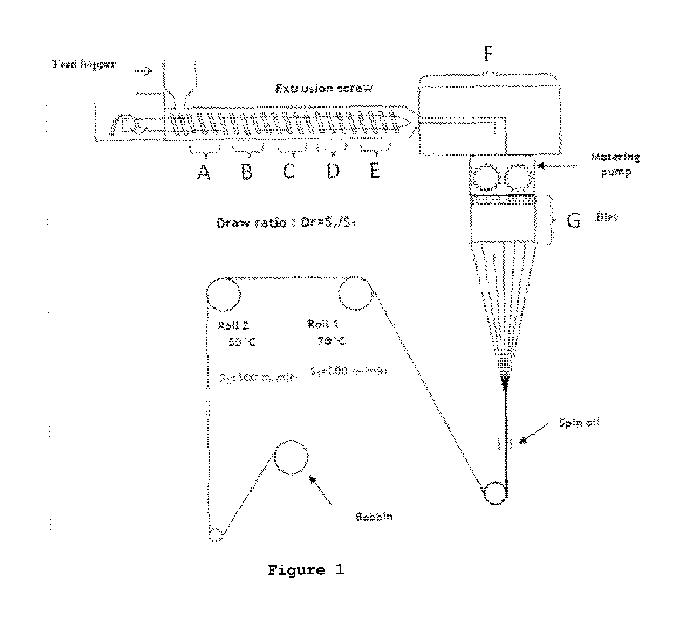

The fibres are produced in a spinning system, as shown in fig. 1. The use of fibres brings several advantages: the surface to volume ratio can be optimized by using several fibres in bundles, thus reducing the response time delay in resistivity measurements, the fibres can be included in smart textile, they can easily be shaped in particular geometrical forms, etc.

The compatibility of the polymer blend has an impact on the spinnability of the biphasic systems. More particularly, the adhesion between both phases improves the spinnability of the blend. This adhesion can be achieved either by the selection of intrinsically adhering pairs of polymers, or by addition of a compatibilizer in one of the polymer phases. Examples of compatibilizers are maleic anhydride grafted polyolefins, ionomers, bloc copolymers comprising a bloc of each phase, etc. This cohesion has also an impact on the blend morphology.

To enable the co-continuity of phases, the ratio of viscosities between the two phases of the biphasic system is preferably close to 1. The other parameters determining the co-continuity are the nature of the polymers (viscosities, interfacial tension and the ratio of these viscosities), their volume fractions and the processing conditions.

## Examples

The examples presented are related to blends comprising:

- Poly($\varepsilon$-caprolactone) (PCL), polyethylene oxide (PEO), polylactic acid (PLA) and polymethyl metacrylate (PMMA) as the first polymer phase;
- polypropylene (PP) and polyamide 12 (PA12) as the second polymer phase;
- Carbon Nanotubes (CNT)

PCL, namely CAPA 6800 from Solvay, is a biodegradable polymer with a relatively low melting temperature of about 60°C. The polyethylene oxide was provided by Sima Aldrich, the grade name was PEO 181986. The PMMA used was VQ101S provided by Rhöm. PLA was the grade L-9000 commercialised by Biomer.

PP of the type H777-25R from DOW was chosen. PA12 was Grilamid L16E from EMS-Chemie. These PP and PA12 are spinning types and should lead to a good spinnability of the blends.

Composites of these polymers with various weight contents of carbon nanotubes (CNT) from Nanocyl were prepared with various weight fractions. Carbon nanotubes are multiwall carbon nanotubes with a diameter between 5 and 20 nm, preferably between 6 and 15 nm, and a specific surface area between 100 $m^2$/g and 400 $m^2$/g.

The production of the fibres was done in a two step process. In a first step, the carbon nanotubes were dispersed in the first polymer in a twin-screw compounding extruder. The obtained extrudates are then pelletized and dry blended with the second polymer.

The obtained dry blend was then fed in the hopper of a single screw extruder, feeding a spinning die as represented in fig. 1. The temperatures in the various zones corresponding to fig. 1 are summarised in table 1. The temperatures were fixed for a given second polymer phase.

Table 1 Temperatures in °C in the different extrusion zones corresponding to fig.1

| First polymer | A | B | C | D | E | F | G |
|---|---|---|---|---|---|---|---|
| PP | 180 | 190 | 200 | 210 | 230 | 230 | 230 |
| PA12 | 180 | 185 | 190 | 195 | 200 | 200 | 200 |

The composition of the conductive polymer composites (CPC) prepared for further experiments are detailed in Table 2.

Table 2: CPC compositions used in co-continuity and conductivity experiments.

| | polymer blend | polymer phase weight fraction : first polymer/ second polymer | CNT weight fractions in the first polymer phase in wt % |
|---|---|---|---|
| Sample 1 | PCL/PP | 80/20 | 3 |
| Sample 2 | PCL/PP | 70/30 | 3 |
| Sample 3 | PCL/PP | 60/40 | 3 |
| Sample 4 | PCL/PP | 50/50 | 3 |
| Sample 5 | PCL/PA12 | 80/20 | 6 |
| Sample 6 | PCL/PA12 | 70/30 | 6 |
| Sample 7 | PCL/PA12 | 60/40 | 6 |
| Sample 8 | PCL/PA12 | 50/50 | 6 |
| Sample 9 | PMMA/PA12 | 50/50 | 2 |
| Sample 10 | PLA/PA12 | 50/50 | 2 |
| Sample 11 | PEO/PA12 | 50/50 | 3 |
| Sample 12 | PCL/PA12 | 50/50 | 3 |

A melt spinning machine (Spinboy I manufactured by Busschaert Engineering) was used to obtain the multifilament yarns. The multifilament yarns are covered with a spin finish, rolled up on two heated rolls with varying speeds (S1 and S2) to regulate the drawing ratio. The theoretical drawing of multifilament is given by the ratio DR = S2/S1. During the fibre spinning, the molten polymer containing nanotubes is forced through a die head with a rather low diameter (400 $\mu$m or 1.2 mm depending on the polymer) and through a series of filters. Several parameters were optimized during the process to obtain spinnable blends. These parameters were mainly the temperature of the heating zones, the speed of the volume metering pump and the roll speed.

**Determination of PCL phase continuity by selective extraction**

An extended study of the co-continuity of the PP/PCL and PA12/PCL blends has been performed. The selective extraction of one phase provides a good estimation of the co-continuity of a mixture. This is achieved by the dissolution of PCL into acetic acid, this solvent having no effect on PA12 and PP. If the mixture has a nodular structure, PCL inclusions will not be affected by the solvent, so they are not dissolved. The percentage of the PCL phase continuity is then deduced by weight loss measurement.

To remove PCL, fibres of each blend were immersed in acetic acid for 2 days at room temperature. The extracted strands were then rinsed in acetic acid and dried at 50°C to remove the acetic acid. After repeating the extraction process several times, the specimen weight converges toward a constant value.

The phase continuity was calculated using the ratio of the soluble PCL part to the initial PCL concentration in the blend, where the dissolvable PCL part is the weight difference of the sample before and after extraction.

The PCL part in the blend is calculated using the following equation:

```
% Continuity of PCL = ((Weight PCLinitial - Weight
PCLfinal) / Weight PCLinitial )* 100%
```

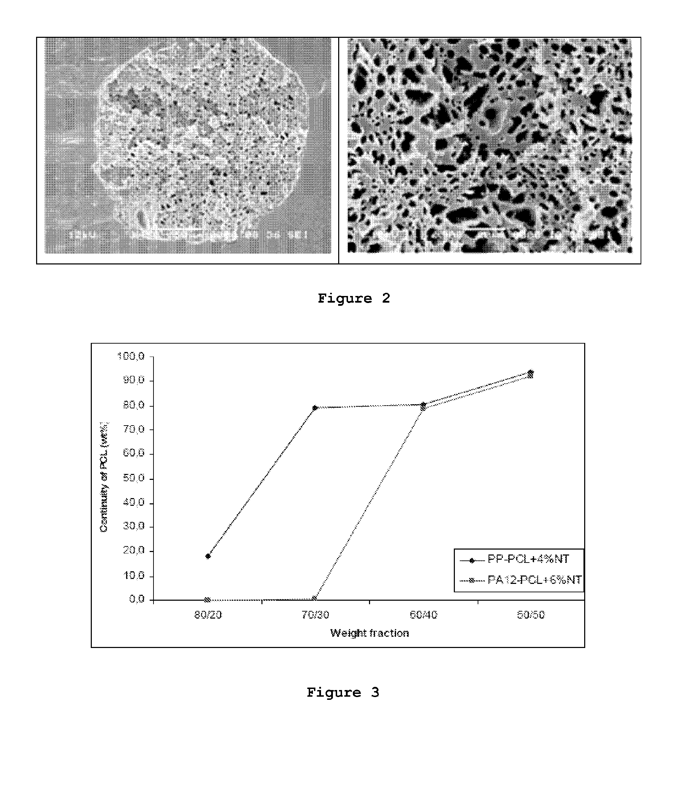

The results are represented in fig. 3. It can be seen in that figure that the continuity of PCL is reached around 40% PCL in PA12 and 30% PCL in PP.

**Vapour sensing**

In the so-called static mode of analysis, the desired amount of solvent molecules to analyse is introduced in a closed chamber. In saturated conditions the solvent is present in a liquid state in the bottom of the chamber. The vapour sensing behaviours of several samples were investigated for toluene, styrene, trichloromethane, THF, water and methanol vapour. Electrical resistance measurements were performed with a Keithley multimeter 2000 at room temperature. Each sample was exposed to saturated organic vapours for 300 s and then moved into a dessicator with dried air atmosphere. Those cycles were repeated three times. The resistance of the fibre was measured automatically every 10s. The relative amplitude was then defined as (R- R0)/ R0, where R0 is the initial resistance of the composite (i.e. when exposed to air atmosphere).

In the so-called dynamic mode, electrochemical properties of the composite fibres comprising CNTs were investigated by recording their electrical responses when submitted to 15 min of successive cycles of nitrogen and vapour streams. The dynamic system consisting in mass flow controllers, solvent bubblers and electrical valves is controlled by the LabView software. Bubbling $N_2$ gas in liquid solvent provided a saturated vapour stream, which was in turn diluted by a second $N_2$ flow to the desired concentration at room temperature. The design of the device allows to keep constant the total flow rate $Q_v$ ($cm^3$. $min^{-1}$), while the analyte flow rate is adapted to investigate the effect of the analyte content. Electrical characteristics of the CPC transducer were recorded with a KEITHLEY 6517A multimeter. Samples were placed in a chamber of 9 cm $\times$ 3 cm $\times$ 3.5 cm.

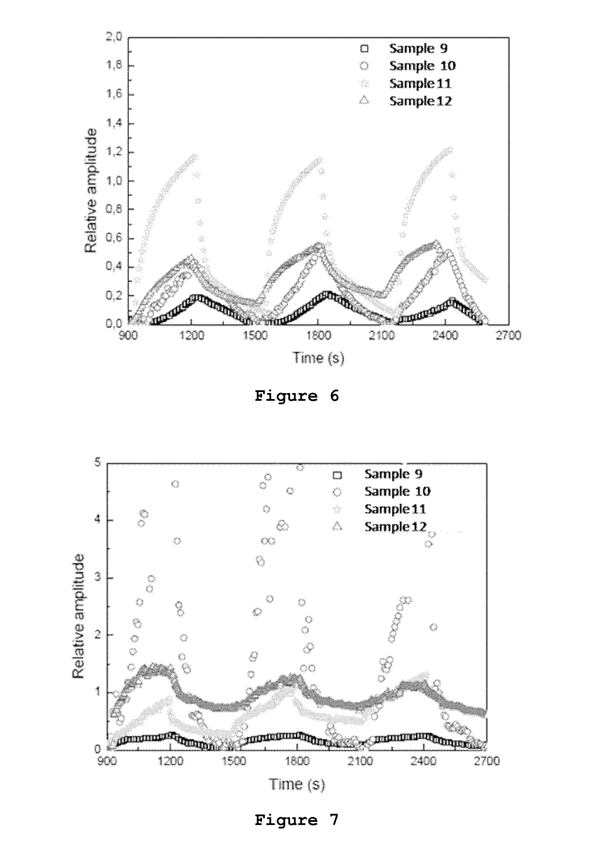

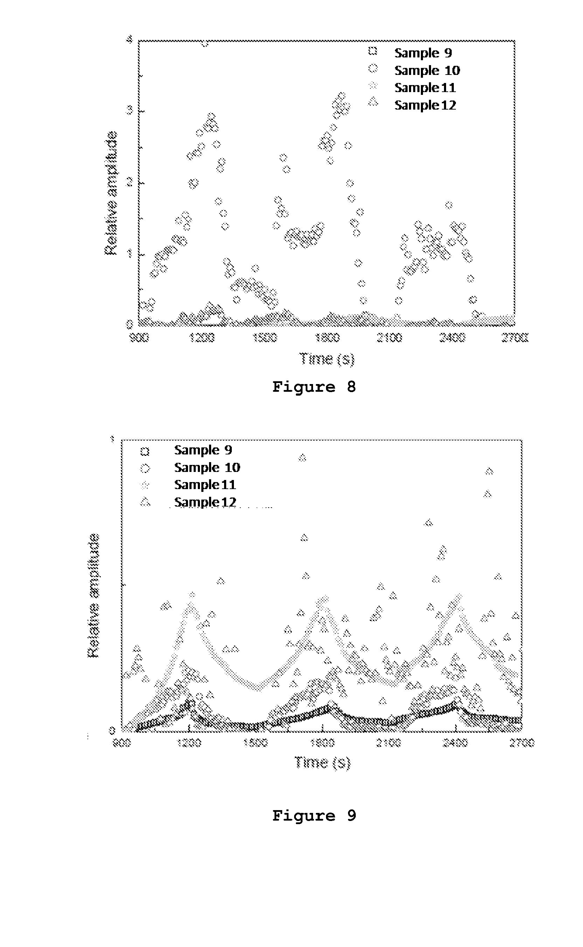

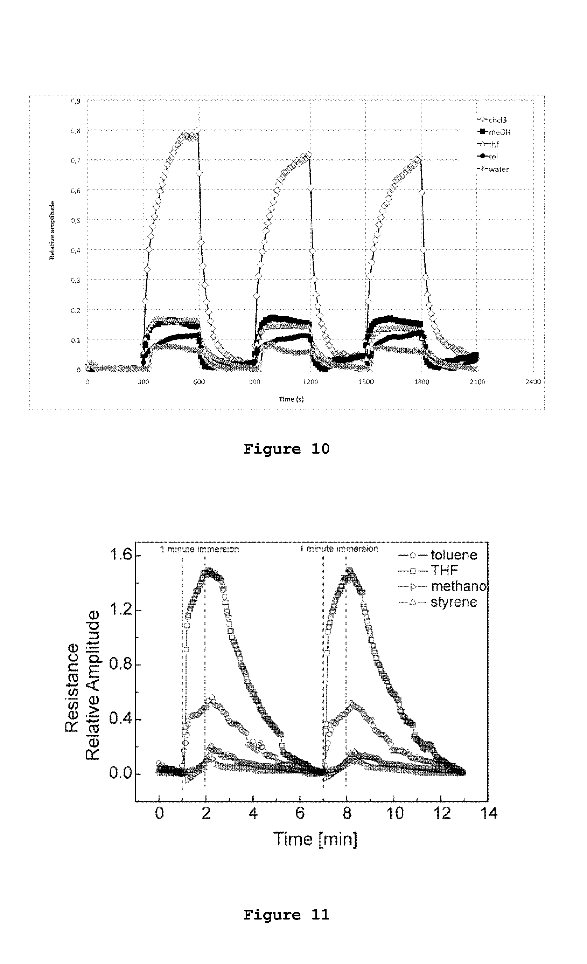

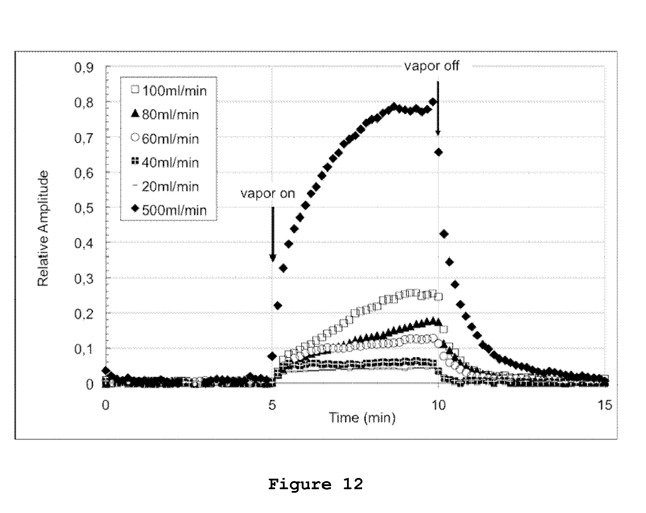

The relative amplitudes obtained with the different samples and different solvents are represented in fig. 6 to 12. It can be seen in those figures that each particular first polymer phase has a specific sensitivity towards a particular solvent. For example, PLA shows a better sensitivity to styrene vapour and PEO shows a better sensitivity to methanol vapour. Those differences can advantageously be used in vapour sensing devices comprising several sensing fibres for improving the selectivity of the devices towards particular chemicals.

**Claims**

1. Fibre-based electrochemical sensor for the detection of at least one chemical analyte vapour in a gaseous environment comprising at least one type of composite fibres, said type of composite fibres comprising a co-continuous phase blend with a specific association of a first and a second continuous polymer phase, the first polymer phase being sensitive to the chemical analyte vapour to be detected in use, wherein said first polymer phase comprises a dispersion of carbon nanotubes at a concentration above the percolation threshold and wherein the chemical analyte is soluble in said first polymer phase.

2. Fibre-based electrochemical sensor according to claim 1, wherein the Flory-Huggins interaction parameter between the chemical analyte and the first polymer phase is smaller than 5,64 [$J^{1/2}$ $cm^{-3/2}$].

3. Fibre-based electrochemical sensor according to claim 1 or 2, wherein said carbon nanotubes are multiwall carbon nanotubes with a specific surface area between 100 and 400 $m^2/g$.

4. Fibre-based electrochemical sensor according to claim 1, 2 or 3, wherein said first polymer phase is selected from the group consisting of polycaprolactone, polylactic acid, polyethylene oxide and polymethyl metacrylate.

5. Fibre-based electrochemical sensor according to any of the previous claims, wherein said at least one chemical analyte is selected from the group consisting of aromatic solvents, preferably styrene and toluene.

6. Fibre-based electrochemical, sensor according to any of the previous claims wherein said at least one chemical analyte is selected from the group consisting of alcohols, preferably methanol.

7. Fibre-based electrochemical sensor according to any of the previous claims, wherein said at least one chemical analyte is selected from the group consisting of chlorinated solvents, preferably trichloromethane.

8. Fibre-based electrochemical sensor according to any of the previous claims, wherein said second polymer phase is a polyolefin, preferably polyethylene or polypropylene.

9. Fibre-based electrochemical sensor according to any of the previous claims, wherein said second polymer phase is polyamide.

10. Fibre-based electrochemical sensor according to any of the previous claims, wherein the first polymer phase is polycaprolactone, for the detection, in use, of tetrahydrofurane.

11. Fibre-based electrochemical sensor according to any of the previous claims, wherein the first polymer phase is polylactic acid for the detection, in use, of Styrene.

12. Fibre-based electrochemical sensor according to any of the previous claims, wherein the first polymer phase is polyethylene oxide for the detection, in use, of methanol.

13. Fibre-based electrochemical sensor according to any of the previous claims, wherein the first polymer phase is polymethyl metacrylate for the detection, in use, of trichloromethane.

14. Device for measuring the concentration of at least one chemical analyte comprising a fibre-based electrochemical sensor according to any of the claims 1 to 13.

15. Device for measuring the concentration of at least one chemical analyte comprising several different fibre-based electrochemical sensors according to any of the claims 1 to 13.

16. Method for detecting at least one chemical analyte comprising the steps of:

- providing a fibre-based electrochemical sensor according to any of the claims 1 to 13;
- measuring the initial electrical conductivity of the fibre-based sensor;
- bringing said fibre-based sensor into contact with at least one chemical analyte to induce a modification of the electrical conductivity of the fibres;
- measuring the modification of the resulting electrical conductivity of said fibre-based sensor and correlating said modification with the identification of the chemical analyte to be detected.

Figure 1

Figure 2

Figure 3

Figure 4

Figure 5

**Figure 6**

**Figure 7**

Figure 8

Figure 9

Figure 10

Figure 11

Figure 12

Europäisches
Patentamt

European
Patent Office

Office européen
des brevets

**EUROPEAN SEARCH REPORT**

Application Number

EP 09 17 8381

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | LU J ET AL: "Thermo- and chemo-electrical behaviour of carbon nanotube filled co-continuous conductive polymer nanocomposites (CPC) to develop amperometric sensors" MATERIALS RESEARCH SOCIETY SYMPOSIUM PROCEEDINGS, US, vol. 1143, 1 December 2008 (2008-12-01), pages 53-58, XP009133196 ISSN: 0272-9172 * page 53, paragraph 2 - page 55, paragraph 1 * * page 56, paragraph 3 - page 57, paragraph 2; figure 5 * | 1-5, 8-10, 14-16 | INV. G01N33/00 G01N27/12 |
| Y | US 2009/101501 A1 (TAO XIAO-MING [CN] ET AL) 23 April 2009 (2009-04-23) * abstract * * paragraphs [0023] - [0025], [0027]; figure 1; examples 1-6 * | 1-5, 8-10, 14-16 | |
| Y | US 6 315 956 B1 (FOULGER STEPHEN H [US]) 13 November 2001 (2001-11-13) * abstract * * column 9, line 24 - column 11, line 67; figures 1-2; examples 1-4 * * column 1, lines 56-66 * | 1-16 | TECHNICAL FIELDS SEARCHED (IPC) G01N |
| Y | WO 2008/109968 A2 (NANOCYL S A [BE]; UNIV BRETAGNE SUD [FR]; FELLER JEAN-FRANCOIS [FR]; C) 18 September 2008 (2008-09-18) * paragraphs [0015] - [0017], [0025], [0027], [0029] - [0031], [0033] - [0034]; figures 1-2,5-6,8; tables 1,2 * | 1-16 | |

-/--

The present search report has been drawn up for all claims

| Place of search | Date of completion of the search | Examiner |
|---|---|---|
| Munich | 7 May 2010 | Lazar, Zala |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 09 17 8381

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | EP 1 803 763 A1 (UNIV RICE WILLIAM M [US]) 4 July 2007 (2007-07-04) * paragraphs [0011] - [0012] * ----- | 1-16 | |
| Y | US 2005/000830 A1 (GLATKOWSKI PAUL J [US] ET AL GLATKOWSKI PAUL J [US] ET AL) 6 January 2005 (2005-01-06) * paragraphs [0032], [0052] * ----- | 16 | |

TECHNICAL FIELDS
SEARCHED        (IPC)

The present search report has been drawn up for all claims

| Place of search | Date of completion of the search | Examiner |
|---|---|---|
| Munich | 7 May 2010 | Lazar, Zala |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

........................................................................................

& : member of the same patent family, corresponding
    document

EPO FORM 1503 03.82 (P04C01)

**EP 2 336 765 A1**

## ANNEX TO THE EUROPEAN SEARCH REPORT
## ON EUROPEAN PATENT APPLICATION NO.

EP 09 17 8381

This annex lists the patent family members relating to the patent documents cited in the above-mentioned European search report.
The members are as contained in the European Patent Office EDP file on
The European Patent Office is in no way liable for these particulars which are merely given for the purpose of information.

07-05-2010

| Patent document cited in search report | | Publication date | Patent family member(s) | | Publication date |
|---|---|---|---|---|---|
| US 2009101501 | A1 | 23-04-2009 | NONE | | |
| US 6315956 | B1 | 13-11-2001 | CA | 2300722 A1 | 16-09-2000 |
| WO 2008109968 | A2 | 18-09-2008 | NONE | | |
| EP 1803763 | A1 | 04-07-2007 | EP | 1818358 A1 | 15-08-2007 |
| US 2005000830 | A1 | 06-01-2005 | NONE | | |

**EP 2 336 765 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5417100 A
- US 5698089 A
- US 5672297 A
- US 7342479 B
- US 6315956 B